# EUROPEAN PATENT SPECIFICATION

(11) **EP 2 926 812 B1**
(45) Date of publication and mention of the grant of the patent: **06.03.2019**
(21) Application number: 13858573.2
(22) Date of filing: 27.11.2013
(51) Int. Cl.: A61K 31/357, A61K 9/19, A61K 47/40, A61P 35/02, A61K 9/00

(54) **USE OF ARTEMETHER IN PREPARATION OF DRUG FOR TREATING LEUKEMIA**
VERWENDUNG VON ARTEMETHER BEI DER HERSTELLUNG EINES ARZNEIMITTELS ZUR BEHANDLUNG VON LEUKÄMIE
UTILISATION D'ARTÉMÉTHER DANS LA PRÉPARATION D'UN MÉDICAMENT DESTINÉ AU TRAITEMENT DE LA LEUCÉMIE

(30) Priority: 29.11.2012 CN 201210499332
(43) Date of publication of application: 07.10.2015
(73) Proprietor: KPC Pharmaceuticals, Inc., Kunming, Yunnan 650106 (CN)
(72) Inventor: WANG, Min, Yunnan 650106 (CN); ZHAO, Junming, Yunnan 650106 (CN); TIAN, Zheng, Yunnan 650106 (CN); CHEN, Yunjian, Yunnan 650106 (CN); LIU, Yidan, Yunnan 650106 (CN); YANG, Zhaoxiang, Yunnan 650106 (CN); YANG, Xujuan, Yunnan 650106 (CN); ZHU, Ze, Yunnan 650106 (CN)
(74) Representative: Potter Clarkson
(86) International application number: PCT/CN2013/087889
(87) International publication number: WO 2014/082569

(56) References cited:
- WO-A1-2004/075921
- CN-A- 1 520 823
- CN-A- 101 125 127
- CN-A- 101 732 250
- SINGH NARENDRA P ET AL: "Case report of a pituitary macroadenoma treated with artemether", 1 December 2006 (2006-12-01), INTEGRATIVE CANCER THERAPIES, SAGE PUBLICATIONS, THOUSAND OAKS, CA, US, PAGE(S) 391 - 394, XP009189828, ISSN: 1534-7354 * the whole document *

## Description

### TECHNICAL FIELD

The present invention relates to the field of medicine, and in particular, to use of artemether in the preparation of a medicament for treating leukemia.

### BACKGROUND OF THE INVENTION

Leukemia is a malignant disease with abnormal hematopoietic stem cells, in which the leukemia cells in cloning lost their ability to further differentiate into mature cells and thus remain in different stages of cell development. In bone marrow and other hematopoietic tissues, a large number of leukemia cells proliferate, accumulate and infiltrate into other organs and tissues, thereby inhibit the normal hematopoiesis. The clinical manifestations of leukemia include the symptoms of anemia, bleeding and various infiltrated organs. This disease is the leading malignant disease in youngsters, but the cause is still not fully understood. Viral may be the major pathogenic factor, whereas many other factors such as radiation, chemical poison or drug, genetic diathesis and the like may be pathogenic cofactors. According to the types of proliferating cells, the disease may be divided into three categories: granulocytic, lymphatic and monocytic leukemia.

Researches in recent years show that anti-malarial drug, artemisinin, and its derivatives exhibit a significant antitumor activity, thus studies on their pharmacological effects have received wide attention. Artemether is one of the main derivatives of artemisinin, and exhibits a better antimalarial activity than artemisinin. More and more experiments in recent years have demonstrated that artemisinin-based drugs have a more wide pharmacological effects, and it is more important that they can selectively inhibit and/or kill various tumor cells, have very little side effects, and even do not result in drug tolerance. The antitumor activity of artemisinin-based drugs have been confirmed in multiple aspects, and the main action mechanism thereof is as the follows:
1. Cell damage mediated by iron ion: Malignant tumor cells have higher Fe content than normal cells. Artemisinin-based drugs can form endoperoxide bridge by mediation of iron ion, and produce reactive oxygen species (ROS) and carbon-centered free radicals, wherein the latter will cause large molecular damage and death of cell.
2. Cell cycle arrest: Artesunate can significantly reduce cell cycle of Kaposi's sarcoma, and dihydroartemisinin may arrest human breast cancer MCF-7 cell in the G0+G1 phase.
3. Induce apoptosis: Artesunate can induce apoptosis of Kaposi's sarcoma in a dosage-dependent manner, but cannot induce apoptosis of normal cells. Artemisinin can induce apoptosis of human erythroleukemia K562 cells by reducing transmembrane potential.
4. Anti-angiogenic effect: Artemisinin-based drugs can prevent the neovascularization of tumor by inhibiting the expression of VEGF and KDR/flk-1.
5. Artemisinin-based drugs can regulate the expression of tumor-associated genes, and thereby achieve antitumor activity.
6. Others: For example, artesunate has the ability against multidrug resistance. Artemisinin can inhibit the synthesis of nuclear factor NF-κB in astrocytoma T67 cell. Furthermore, artemether may induce damage and swell of mitochondria, fracture, reduction and disappearance of crista, as well as inhibit the activity of complexes I and IV of mitochondrial respiratory chain, and thereby result in cell death.

WO 2004/075921 describes a method for preparing pharmaceutical compositions comprising artemisinin or derivatives thereof and a cyclodextrin, obtained by the methods described therein. The pharmaceutical compositions may be used in the treatment of diseases including malaria, cancer, babesiosis, shistosomiasis, and fungal, viral and/or bacterial infections.

N.P. Singh et al. 2006, Integr. Cancer. Ther., 5(4), 391-394, describes the case of a male patient with pituitary macroadenoma treated with the artemisinin analogue artemether. The artemether was administered orally to the patient over a period of 12 months with the tumour remaining constant in size and CT scans showing a reduction in the tumours density along with related symptoms and signs being resolving significantly as therapy progressed.

### SUMMARY OF THE INVENTION

The object of the present invention is to provide artemether for use in treating leukemia.

Preferably, the drug is an artemether injection or lyophilized powder for injection.

The artemether injection or lyophilized powder for injection of the present invention consists of artemether and a water-soluble cyclodextrin derivative in a weight ratio of 1:30-70. The water-soluble cyclodextrin derivative comprises one or several of alpha-cyclodextrin derivative, beta-cyclodextrin derivative, gamma-cyclodextrin derivative and the like with various substitution degrees. The beta-cyclodextrin derivative comprises one or several of methyl-beta-cyclodextrin, hydroxypropyl-beta-cyclodextrin, thioether-beta-cyclodextrin, and sulfobutyl ether-beta-cyclodextrin, or a combination thereof.

As described herein, the preparation method of the artemether injection or lyophilized powder for injection comprises the following steps: artemether is pulverized in a low-temperature pulverizer at 0°C-10°C, and the pulverized artemether is added into an aqueous solution of a water-soluble cyclodextrin derivative at 50°C-90°C, stirred to dissolve artemether, then treated or lyophilized according to a conventional process of an injection to obtain a finished product.

The artemether injection or lyophilized powder for injection of the present application is prepared by micronization technology at low temperature (0°C-10°C), avoiding the degradation of artemether caused by local excessive temperatures in the conventional pulverization technique. The artemether is divided into different groups according to the particle size, and tested, and the result shows that at the same temperature, the dissolution rate of artemisinin derivatives reduce as the particle size increases. When the particle size is greater than 315 gm, the preparation temperature is 70°C-90°C. The micronized artemether drug has a particle size in the range of 0.5 µm ∼ 0 µm, optimal in the range of 1.0 µm ∼ 10 µm. The increase of the preparation temperature facilitates the dissolution of artemether, and the optimal preparation temperature is 50 °C ∼60 °C, which temperature may reduce the decomposition, oxidation and the like of the drug.

The inventors have demonstrated through numerous experiments that, artemether lyophilized powder for injection administered at a high dosage of 100 mg/kg for 3 weeks (5 doses per week, a total of 15 doses) exhibits significant inhibition effect on the growth of K562 nude mice transplanted tumor model, and the inhibition ratios reached 68%∼80% (P< 0.01) after 3-week administration. For artemether lyophilized powder for injection administered at a middle dosage of 60 mg/kg for 3 weeks (a total of 15 doses and 21 doses, respectively), the inhibition ratios reached 31% and 56% (P>0.05 and P<0.01), respectively. When artemether lyophilized powder for injection was administered at a low dosage of 40 mg/kg for 3weeks, no significant inhibition was found for 15 doses, and the inhibition ratio was 55% (P<0.01) for 21 doses. When artemether was administered at dosages of 100mg/kg, 60 mg/kg and 40 mg/kg, respectively, for 3 weeks (5 days of administration and 2 days rest per week, a total of 15 doses), no animal was found death due to drug accumulation.

The above results shows that, artemether injection or lyophilized powder for injection exhibit significant inhibition effect on the growth of K562 nude mice transplanted tumor model at the two dosages of 100 mg/kg and 60 mg/kg, and the inhibition ratios may reach 60%∼80% (P<0.01). The result shows that artemether has significant therapeutic effect on leukemia, and has an inhibition ratio close to that of cyclophosphamide as positive control, and thus is a new potential anti-tumor drug.

### BRIEF DESCRIPTION OF THE DRAWINGS

Figure 1 shows the tumor sizes in tumor-bearing animals in each group 4 weeks after administration in pre-trial.
Figure 2 shows the tumor sizes of animals in each group 4 weeks after administration in pre-trial.
Figure 3 shows the tumor sizes in tumor-bearing animals in each group 4 weeks after administration in experiment 1.
Figure 4 shows the tumor sizes of animals in each group 4 weeks after administration in experiment 1.
Figure5 shows the tumor sizes in tumor-bearing animals in each group 4 weeks after administration in experiment 2.
Figure 6 shows the tumor sizes of animals in each group 4weeks after administration in experiment 2.

### DETAILED DESCRIPTION OF THE INVENTION

The present invention discloses use of artemether in the preparation of a medicament for treating leukemia. Those skilled in the art may modify properly the parameters in the process according to the disclosure herein, so as to achieve the present invention. It should be noted that, all the similar replacements and modification are apparent for those skilled in the art, and thus are contemplated to be included in the present invention. The use of the present invention has been described by examples.

Now, the present invention will be further illustrated in detail in combination with examples in order to render those skilled in the art to understand the present invention better.

### Example: Therapeutic effect of artemether on leukemia.

The inventors observed the therapeutic effect of artemether at different dosages on K562 nude mice transplanted tumor. The result shows that artemether administered at high dosages of 100 mg/kg and 60 mg/kg for 15 doses can significantly inhibit the growth of the tumor in the tumor- bearing mice; artemether administered a dosage of 40 mg/kg for 21 doses also exhibits a significant inhibitory effect. The experiment result shows that artemether can inhibit the growth of human leukemia K562 nude mice transplanted tumor, and thus has a significant antitumor activity.
**1. Purposes of study:** to establish a K562 nude mice transplanted tumor model in a nude mice and observe the inhibitory effect of artemether on the growth of nude mice transplanted tumor.

### 2. Experimental material:

2.1 test drug: artemether lyophilized powder for injection, lot number: 20090903. The preparation method comprises the following steps: artemether is pulverized in a low-temperature pulverizer at 0°C-10°C, and the pulverized artemether is added into an aqueous solution of a water-soluble cyclodextrin derivative at 50°C-90°C, stirred to dissolve artemether, then treated or lyophilized according to a conventional process of an injection to obtain a finished product; wherein the weight ratio of artemether and the water-soluble cyclodextrin derivative is 1:30-70.
2.2 control:
2.2.1 negative control: 20% aqueous solution of excipient is used as negative control.
2.2.2 positive control: cyclophosphamide (LIAN HUA Pharmaceutical Factory, Shanghai, China), lot number: 10022321.
2.3 vehicle: 20% aqueous solution of excipient.
2.4 K562 cell: primary K562 cell is derived from bone marrow of a patient having leukemia, introduced by Blood Research Institute from abroad, stored and passaged in Pathologic Cell Bank; cultured in RPMI1640 medium (comprising 10% fetal bovine serum).

### 2.5 Experimental equipment

2.5.1 conventional cell culture equipment.
2.5.2 cesium source irradiation unit: NORDION Corp., Canada.
2.5.3 III-class (SPF) animal laboratory.
2.6 experimental animal: Balb/c nude mice, 4-5 weeks old, weight: 17-20 g, gender: half male and half female. Experimental Animal Institute of the Chinese Academy of Medical Sciences, certification number of experimental animals: SCXK (JING) 2005-0013.
2.7 environmental condition and breeding and management: experiments of nude mice are performed under SPF conditions in Blood Research Institute of the Chinese Academy of Medical Sciences. Laboratory animal facility license number: SYXK(JIN) 2009-0002. Feedstuff: autoclaved mouse feed, manufactured by Vitalriver Technologies. Ltd. standard: GB 14924.2-2001; lot number: 2009110. Water: sterile water purified by reverse osmosis. Padding: mixed small shavings of fir wood and cypress wood are sieved to remove the dust, and autoclaved for use. Feeding: male and female mice are hosed in different cages, 5 animals per cage, feed is delivered according to consumption, and water is available *ad libitum.* The cages are autoclaved after cleaning, and the cages and padding are replaced every 2 weeks. The house is sterilized alternately by peracetic acid and bromo-geramine twice a week.

### 3. Experimental procedures

3.1 **pre-trial:**
3.1.1 Balb/c nude mice were subjected to 3.0Gy whole-body irradiation, after subcutaneous inoculation of primary K562 cells. About 4 weeks after inoculation, tumor masses in growing were removed and transplanted into normal mice, and this process was repeated for 2∼3 passages to establish high oncogenic K562 cell line.
3.1.2 Nude mice were subjected to 3.0Gy whole-body irradiation, after subcutaneous inoculation of the K562 cells. One week after inoculation, nude mice with visible tumors were divided into the following 4 groups each consisting of 6 animals. The following dosages were administered for 3 continuous weeks, with 7 doses per week, a total of 21 doses.

Model control group: intraperitoneal injection of 20% aqueous solution of excipient at 0.2 ml/animal.

High dosage group: intraperitoneal injection of artemether at 150 ml/kg.

Middle dosage group: intraperitoneal injection of artemether at 100 ml/kg.

Low dosage group: intraperitoneal injection of artemether at 50 ml/kg.

### 3.2 Experiment 1:

Nude mice were subjected to a 3.0Gy whole-body irradiation, after subcutaneous inoculation of the K562 cells. One week after inoculation, nude mice with visible tumors were divided into the following 5 groups each consisting of 6 animals. The following dosages were administered for 3 continuous weeks, with 5 doses per week, a total of 15 doses.

Model control group: intraperitoneal injection of 20% aqueous solution of excipient at 0.2 ml/animal.

Positive control group: intraperitoneal injection of cyclophosphamide at 10 ml/kg.

High dosage group: intraperitoneal injection of artemether at 100 ml/kg.

Middle dosage group: intraperitoneal injection of artemether at 60 ml/kg.

Low dosage group: intraperitoneal injection of artemether at 40 ml/kg.

### 3.3 Experiment 2:

Nude mice were subjected to a 3.0Gy whole-body irradiation, after subcutaneous inoculation of the K562 cells. One week after inoculation, nude mice with visible tumors were divided into the following 5 groups each consisting of 6 nude mice.

Model control group: intraperitoneal injection of 20% solution of excipient at 0.2ml/animal. The dosage was administered for 3 weeks, with 7 doses per week, a total of 21 doses.

Positive control group: intraperitoneal injection of cyclophosphamide at 10 ml/kg. The dosage was administered for 3 weeks, with 7 doses per week, a total of 21 doses.

High dosage group: intraperitoneal injection of artemether at 100 ml/kg. The dosage was administered for 3 weeks, with 5 doses per week, a total of 15 doses.

Middle dosage group: intraperitoneal injection of artemether at 60 ml/kg. The dosage was administered for 3 weeks, with 7 doses per week, a total of 21 doses.

Low dosage group: intraperitoneal injection of artemether at 40 ml/kg. The dosage was administered for 3 weeks, with 7 doses per week, a total of 21 doses.

### 4. Experimental results

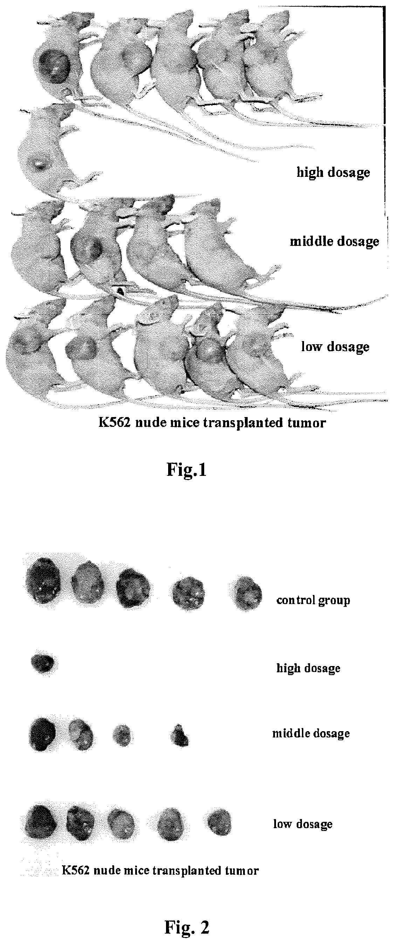

### 4.1 Results of pre-trial are summarized in tables 1-3 and figures 1-2.

**Table 1 Dynamic observation results of tumor volumes for each group of animals at different time points after administration (unit: mm³).**

| time | group | the number of Animals | tumor volume | inhibition ratio | statistical result |
|---|---|---|---|---|---|
| 1 week after administration | Control group | 6 | 106.6±70.22 | | |
| | High dosage group | 1 | 55.3 | | |
| | Middle dosage group | 5 | 93.6±43.6 | | |
| | Low dosage group | 6 | 174.9±74.7 | | |
| 2 weeks after administration | Control group | 6 | 363.3±230.78 | | |
| | High dosage group | 1 | 136.8 | | |
| | Middle dosage group | 5 | 219.9±110.83 | | |
| | Low dosage group | 6 | 535.5±253.42 | | |
| 3 weeks after administration | Control group | 5 | 1766.9±392.98 | | |
| | High dosage group | 1 | 454.1 | | |
| | Middle dosage group | 4 | 524.6±265.57 | 70.3% | P<0.05 |
| | Low dosage group | 5 | 997.1±327.71 | 43.6% | |
| 4 weeks after administration | Control group | 5 | 3017.0±536.2 | | |
| | High dosage group | 1 | 663.1 | | |
| | Middle dosage group | 4 | 931.8±307.9 | 69.1% | P<0.01 |
| | Low dosage group | 5 | 1672.0±331.0 | 44.6% | P<0.01 |

**Table 2 Comparison of tumor mass in each group 4 weeks after administration**

| group | tumor mass (mg) | inhibition ratio | statistical result |
|---|---|---|---|
| Control group | 2440±646 | | |
| High dosage group | 590 | | |
| Middle dosage group | 857±574.4 | 64.9% | P<0.01 |
| Low dosage group | 1436±281.3 | 41.1% | P<0.01 |

**Table 3 observed apoptosis results of tumor cells in each group**

| group | apoptosis ratio(%) |
|---|---|
| Control group | 8.86±6.23 |
| High dosage group | 2.63 |
| Middle dosage group | 2.04±1.58 |
| Low dosage group | 1.37±0.47 |

The effect of the drug on the apoptosis of the tumor cell: from the result of pre-trial (table 3), it appears that the apoptosis ratio in control group is higher than that in the treated group. In fact, this is because the tutor of control group grows more rapidly, has a larger volume, and thus a more significant necrosis. According to our experience, unlike an in vitro test, in an in vivo test, drug inhibits the growth of tumor, and exhibits as a slower growth of tumor and a smaller tumor mass in experimental group. However, cells which are not inhibited by the drug form a tumor mass that are not different much from the tumor mass in control group. Therefore, in a mechanism study, tumor cells *in vivo* should not be used, and an *in vitro* test is preferred.

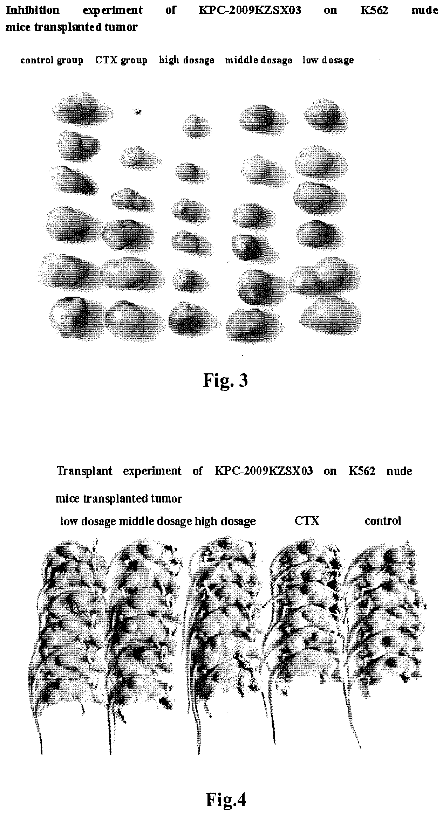

### 4.2 Results of experiment 1 are summarized in tables 4-5 and figures 3-4.

**Table 4 Dynamic observation results of tumor sizes at different time points after inoculation (unit: mm3) n=6.**

| group | 1 week | 2 weeks | 3 weeks | 4 weeks |
|---|---|---|---|---|
| Control group | 56.5±24.3 | 242.2±76.0 | 596.8±309.8 | 1403.2±379.4 |
| Cyclophosphamide group | 48.1±10.9 | 104.9±60.3# | 373.7±319.9 | 799.0±571.3* |
| High dosage group | 55.6±11.4 | 72.9±70.8# | 307.9±223.2* | 489.2±199.9# |
| Middle dosage group | 51.3±4.5 | 127.5±22.3 | 376.7±135.8 | 1035.6±248.5 |
| Low dosage group | 42.0±9.0 | 148.4±75.6 | 432.6±332.5 | 1127.7±142.8 |

| | | | | |
|---|---|---|---|---|
| * indicates P < 0.05 compared with control group; # indicates P < 0.01 compared with control group; | | | | |

**Table 5 Comparison of tumor mass in each group at 4 weeks after inoculation**

| group | Tumor mass(mg) | Inhibition ratio |
|---|---|---|
| Control group | 1073.3±358.0 | |
| Cyclophosphamide group | 651.7±544.3# | 39.2% |
| High dosage group | 340.0±237.6# | 68.3% |
| Middle dosage group | 736.7±247.6 | 31.4% |
| Low dosage group | 1135.0±457.2 | -5.7% |

| | | |
|---|---|---|
| # indicates P < 0.01 compared with control group; | | |

The above results show that, when artemether is administered at dosages of 100mg/kg, 60 mg/kg and 40 mg/kg, respectively, for 3 continuous weeks (5 days dosing and 2 days rest per week, a total of 15 doses), no animal is found dead due to drug accumulation.

At 1 week (5 doses) after administration, the tumor growth rate of High dosage group is inhibited, and tumor volume is significantly smaller than that of control group. Up to the end of administration, the tumor growth (tumor mass) of High dosage group is inhibited up to 68% or more. Middle dosage group is inhibited by 30% or more, but no significant difference in statistical analysis. Low dosage group has no significant effect.

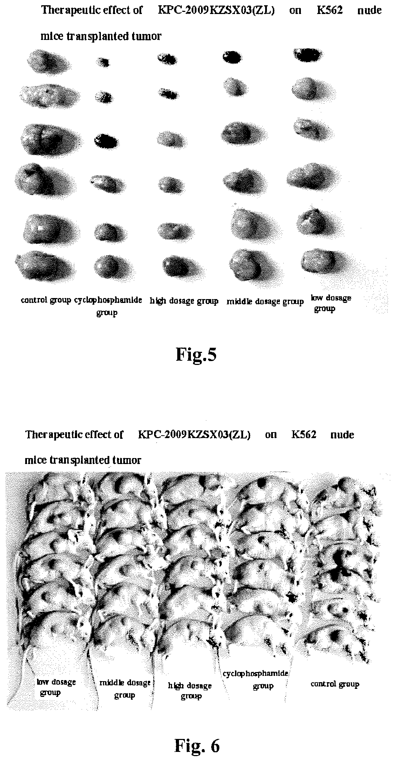

### 4.3 Results of experiment 2 are summarized in tables 6-7 and figures 5-6.

**Table 6 Dynamic observation results of tumor sizes at different time points after inoculation (unit: mm³) n=6.**

| group | 1 week | 2 weeks | 3 weeks | 4 weeks |
|---|---|---|---|---|
| Control group | 31.6±6.62 | 243.6±117.4 | 907.1±154.6 | 1838.8±586.6 |
| Cyclophosphamide group | 30.7±10.9 | 77.3±49.8 | 203.1±94.7* | 327.0±206.8* |
| High dosage group | 33.9±14.4 | 75.5±27.2 | 137.1±101.6* | 291.7±191.5* |
| **Middle dosage** group | 22.7±7.4 | 118.4±129.0 | 201.2±98.9* | 746.6±393.7* |
| **Low dosage** group | 26.7±5.9 | 203.7±127.3 | 297.4±86.6* | 729.2±272.8* |

| | | | | |
|---|---|---|---|---|
| * indicates P < 0.01 compared with control group; | | | | |

**Table 7 Comparison of tumor mass in each group 4 weeks after inoculation**

| group | tumor mass(mg) | inhibition ratio |
|---|---|---|
| Control group | 1450±434.4 | |
| Cyclophosphamide group | 241.7±176.8* | 83.3% |
| High dosage group | 230±153.4* | 84.1% |
| Middle dosage group | 630±418.9* | 56.6% |
| Low dosage group | 645±314.7* | 55.5% |

| | | |
|---|---|---|
| # indicates P < 0.01 compared with control group; | | |

1. The above results show that when artemether is administered at dosages of 100mg/kg, 60 mg/kg and 40 mg/kg, respectively, for 3 continuous weeks (a total of 15 doses for high dosage, a total of 21 doses for each of middle and low dosages), no animal is found dead due to drug accumulation.
2. Upon one week (5-7 doses) after administration, the tumor growth rate of each dosage group is inhibited, and tumor volume is significantly smaller than that of control group. Upon 3 weeks after administration, the tumor growth (tumor mass) of high dosage group is inhibited by 80% or more. Middle and low dosage group is inhibited by 50% or more, but no significant difference (p < 0.01) is observed between the tumor volume and mass for administered group compared with control group.
3. When using middle and low dosages and increasing the times of administration, the therapeutic effects of middle and low dosages are improved without toxicity.

### Conclusion and evaluation

The research results show that, for artemether lyophilized powder for injection administered at High dosage of 100 mg/kg for 3 continuous weeks (5 doses per week, a total of 15 doses) exhibits a very significant inhibition effect on the growth of K562 nude mice transplanted tumor model, and the inhibition ratios can reach 67%∼80% (P<0.01) 3 weeks after administration; for artemether lyophilized powder for injection administered at a **Middle dosage** of 60 mg/kg for 3 continuous weeks (a total of 15 doses and 21 doses, respectively), the inhibition ratios reach 31% and 56% (P>0.05 and P<0.01), respectively; for artemether lyophilized powder for injection administered at a **Low dosage** of 40 mg/kg for 3 continuous weeks, no significant inhibition is found for 15 doses, and the inhibition ratios is 55% (P<0.01) for 21 doses.

The above results show that, artemether exhibits significant inhibition effect on the growth of K562 nude mice transplanted tumor model at the two dosages of 100 mg/kg and 60 mg/kg, and the inhibition ratios can reach 60%∼80% (P<0.01). The result shows that the test drug has a significant therapeutic effect on leukemia, and has an inhibition ratio close to that of cyclophosphamide as positive control, and thus is a new potential anti-tumor drug.

## Claims

1. Artemether for use in treating leukemia.

2. Artemether for use in treating leukemia according to claim 1, **characterized in that** the artemether is formulated for injection or as a lyophilized powder for injection.

3. Artemether for use in treating leukemia according to claim 2, **characterized in that** the artemether injection or lyophilized powder for injection consists of artemether and a water-soluble cyclodextrin derivative in a weight ratio of 1:30-70.

4. Artemether for use in treating leukemia according to claim 3, **characterized in that** the water-soluble cyclodextrin derivative comprises one or several of alpha-cyclodextrin derivative, beta-cyclodextrin derivative and gamma-cyclodextrin derivative with various substitution degrees.

5. Artemether for use in treating leukemia according to claim 4, **characterized in that** the beta-cyclodextrin derivative comprises one of methyl-beta-cyclodextrin, hydroxypropyl-beta-cyclodextrin, thioether-beta-cyclodextrin and sulfobutyl ether-beta-cyclodextrin, or a combination thereof.

## Patentansprüche

1. Artemether zur Verwendung beim Behandeln von Leukämie.

2. Artemether zur Verwendung beim Behandeln von Leukämie nach Anspruch 1, **dadurch gekennzeichnet, dass** der Arthemether zum Injizieren oder als ein lyophilisiertes Pulver zum Injizieren formuliert ist.

3. Artemether zur Verwendung beim Behandeln von Leukämie nach Anspruch 2, **dadurch gekennzeichnet, dass** die Artemetherinjektion oder das lyophilisierte Pulver zum Injizieren aus Artemether und einem wasserlöslichen Cyclodextrinderivat in einem Gewichtsverhältnis von 1:30-70 besteht.

4. Artemether zur Verwendung beim Behandeln von Leukämie nach Anspruch 3, **dadurch gekennzeichnet, dass** das wasserlösliche Cyclodextrinderivat eines oder einige eines Alpha-Cyclodextrinderivats, eines Beta-Cyclodextrinderivats und eines Gamma-Cyclodextrinderivats mit unterschiedlichen Substitutionsgraden umfasst.

5. Artemether zur Verwendung beim Behandeln von Leukämie nach Anspruch 4, **dadurch gekennzeichnet, dass** das Beta-Cyclodextrinderivat ein Methyl-Beta-Cyclodextrin, ein Hydroxypropyl-Beta-Cyclodextrin, ein Thioether-Beta-Cyclodextrin oder ein Sulfobutylether-Beta-Cyclodextrin oder eine Kombination daraus umfasst.

## Revendications

1. Artéméther pour une utilisation dans le traitement de la leucémie.

2. Artéméther pour une utilisation dans le traitement de la leucémie selon la revendication 1, **caractérisé en ce que** l'artéméther est formulé pour une injection ou sous la forme d'une poudre lyophilisée injectable.

3. Artéméther pour une utilisation dans le traitement de la leucémie selon la revendication 2, **caractérisé en ce que** l'artéméther injection ou poudre lyophilisée injectable est composé d'artéméther et d'un dérivé hydrosoluble de la cyclodextrine dans un rapport en poids de 1:30-70.

4. Artéméther pour une utilisation dans le traitement de la leucémie selon la revendication 3, **caractérisé en ce que** le dérivé hydrosoluble de la cyclodextrine comprend un ou plusieurs du dérivé de l'alpha-cyclodextrine, du dérivé de la bêta-cyclodextrine et du dérivé de la gamma-cyclodextrine avec différents degrés de substitution.

5. Artéméther pour une utilisation dans le traitement de la leucémie selon la revendication 4, **caractérisé en ce que** le dérivé de la bêta-cyclodextrine comprend l'une de la méthyl-bêta-cyclodextrine, de l'hydroxypropyl-bêta-cyclodextrine, de la thioéther-bêta-cyclodextrine et de la sulfobutyléther-bêta-cyclodextrine, ou une combinaison de celles-ci.
